# EUROPEAN PATENT SPECIFICATION

(11) **EP 0 915 828 B1**
(45) Date of publication and mention of the grant of the patent: **20.03.2002**
(21) Application number: 97930683.4
(22) Date of filing: 11.07.1997
(51) Int. Cl.: C07C 227/40, C07C 227/42, C07C 229/24

(54) **A PROCESS FOR THE PRODUCTION OF CRYSTALLINE ASPARTIC ACID**
VERFAHREN ZUR HERSTELLUNG VON KRISTALLINER ASPARAGINSÄURE
PROCEDE DE PRODUCTION D'ACIDE ASPARTIQUE CRISTALLIN

(30) Priority: 18.07.1996 IL 11889296
(43) Date of publication of application: 19.05.1999
(73) Proprietor: AMYLUM EUROPE N.V., 9300 Aalst (BE); A.E. STALEY MANUFACTURING COMPANY, Decatur Illinois 62521 (US)
(72) Inventor: EYAL, Aharon, 93380 Jerusalem (IL); CAMI, Pierre, F-80190 Languevoisin (FR); PILON, Robert, F-80190 Nesle (FR)
(74) Representative: Ablewhite, Alan James
(86) International application number: GB9701875
(87) International publication number: WO9803468

(56) References cited:
- EP-A- 0 296 937
- EP-A- 0 683 231
- CH-A- 401 987
- FR-A- 1 591 950
- FR-A- 2 666 996
- CHEMICAL ABSTRACTS, vol. 120, no. 7, 14 February 1994 Columbus, Ohio, US; abstract no. 75649, TERASAWA M. ET AL.: "Separation and recovery of D-malic acid" XP002041968 & JP 05 271 147 A (MITSUBISHI PETROCHEMICAL CO.) 19 October 1993
- CHEMICAL ABSTRACTS, vol. 100, no. 25, 18 June 1984 Columbus, Ohio, US; abstract no. 210423, MITSHUBISHI: "Recovery of amino acids" XP002041969 & JP 58 210 027 A (MITSUBISHI CHEMICAL INDUSTRIES CO., LTD.) 7 December 1983

## Description

The present invention relates to a process for the production of crystalline aspartic acid.

Aspartic acid is an acidic amino acid with a molecular formula of HOOCCH₂CH(NH₃)COO. It is used in products such as the aspartame sweetener and for formation of the biodegradable polymer polyaspartic acid (PAA). The latter could be utilized as a cobuilder or as a sequestrant in detergents, as a superabsorbent polymer and in other applications. The biodegradability of PAA is very attractive, and the potential market is large. It strongly depends, however, on the availability of a low cost aspartic acid and a non-contaminating process for the preparation of aspartic acid.

US patent 3,391,059 describes a process wherein microorganisms capable of converting ammonium maleate directly into aspartic acid (or its salt), are isolated. US patent 4,013,508 describes a process utilizing two different microorganisms. One converts hydrocarbons to fumaric acid, which is then converted by the other to aspartic acid.

The ammonium aspartate formed contains various impurities that could result from the enzyme, the nutrients used, products of said reactions, etc. Currently, it is purified by acidulation through the addition of a stoichiometric amount of a mineral acid, e.g. sulfuric acid, to reach a pH in the range of about the isoelectric point of the aspartic acid. The aspartate ion is converted to aspartic acid in zwitterionic form, which precipitates out of the solution. This solution contains ammonium sulfate as the main component. The presence of the sulfate salt decreases the purity of the precipitating product due to coprecipitation of contaminants, which coprecipitation is induced by the high ionic strength. It also increases the solubility of aspartic acid in the solution, which reduces the yields. Another disadvantage of the process is the consumption of ammonia and sulfuric acid and the formation of a salt that can be used only as a low grade fertilizer (in most cases at the added cost of the energy and equipment needed to crystallize it out of the solution).

US patent 4,560,653, assigned to W.R. Grace, claims a process for preparing L-aspartic acid, wherein a substrate containing fumarate ions is reacted in the presence of an aspartase, the improvement comprising reducing the pH of the aqueous solution resulting from the contact with said aspartase or aspartase-producing microorganism to about 3 to 4 by addition of maleic anhydride, maleic acid or salts thereof, to insolubilize L-aspartic acid while providing maleic acid in the supernatant phase, removing the insoluble L-aspartic acid, isomerization of the maleic acid in the supernatant phase to fumaric acid, adjusting the pH of the supernatant phase to about 8 to 9, and passing the supernatant phase into contact with aspartase or aspartase-producing microoganism.

The process described in US patent 4,560,653 solves the problem of ammonia and sulfuric acid consumption and of finding an outlet for ammonium sulfate through acidulation with maleic acid, but has other drawbacks. The aspartic acid is still precipitated from a solution of high ionic strength, which affects its purity. Maleic acid is also found in the crystals. Aspartic acid is left in the mother liquor. It is not lost if the ammonium maleate formed in the mother liquor on such acidulation is fully converted to ammonium fumarate and recycled. That, however, could not be the case, as no outlet is provided for impurities resulting from the biological conversion of fumarate to aspartate, the chemical conversion of maleate to fumarate and from other sources. Rejection of these impurities requires a significant bleed from the mother liquor, which involves product and reagent losses. Moreover, recycling of a solution containing a significant concentration of aspartic acid could inhibit the biological conversion of fumarate to aspartate.

Another patent, assigned to Rhone Poulenc, suggests acidulation of the ammonium aspartate solution with fumaric to precipitate aspartic acid and to form ammonium fumarate. Adding fumaric acid to the solution of ammonium aspartate instead of maleic acid avoids the costs and contamination related to converting maleate into fumarate in a solution, which is sent to the conversion of fumarate to aspartate. it also provides for using as a raw material fumaric acid produced by fermentation rather than petrochemically. However, it does not solve all other difficulties related to product purity, losses, and to the slowing down of the bioconversion by the recycled aspartic acid.

With this state of the art in mind, there is now provided according to the present invention a process for the production of crystalline aspartic acid comprising the steps of: (a) reacting a feed solution containing anions of a dicarboxylic reagent acid, whereby the reagent acid is converted to aspartate ions, to form a product solution containing a dissociated aspartate salt; (b) acidulating a solution of said dissociated aspartate salt resulting from said reaction in step (a) by contacting it with a cation exchanger, whereby cations of the aspartate salt are adsorbed and protons are released to the solution; (c) separating the aqueous solution from the cation exchanger; (d) crystallizing aspartic acid from said separated solution; (e) regenerating said cation exchanger obtained in step (b) by contacting it with a solution containing the reagent acid or an acidic salt thereof, whereby protons from the solution are adsorbed on the cation exchanger and cations adsorbed in step (b) are released to the solution; and (f) using the solution obtained in step (e) as a constituent of said feed solution.

From the molecular formula of aspartic acid, HOOCCH₂CH(NH₃)COO when in zwitterionic form, it is clear that dicarboxylic acids of four carbon atoms provide a preferred reagent acid for its formation. Among those, the most preferred ones are maleic acid (which could be obtained from maleic anhydride) and fumaric acid.

Aspartase converts ammonium fumarate to L-aspartate. Cultivating an aspartase-producing microorganism in a nutrient medium containing diammonium fumarate results in ammonium aspartate. Alternatively, it can be prepared either by reacting whole cells containing aspartase, or by extracting the enzyme and heating it with diammonium aspartate. A possible improvement is immobilization of the enzyme or enzyme-producing microorganism in or on a support structure.

Enzymatic conversion of fumarate to aspartate, referred to in the following also as bioconversion, typically uses an aqueous feed solution containing mainly diammonium fumarate, and the product is, in fact, ammonium aspartate. Bioconversions are typically conducted at a temperature of about 50 to 60C. The pH in the system is about 8 to 9. In present industrial practice, the fumarate ion concentration in that feed solution is high in order to minimize crystallization losses.

Fixed cell bioconversion works usually at a lower temperature and concentration to enhance stability. As a result, recovery yields in the present process could decrease. This problem is solved by a preferred embodiment of the present invention as explained in the following.

The pKa's of aspartic acid are 1.88, 3.65 and 9.60, and its isoelectric point (pl) is 2.77. The aspartate-containing solution obtained in the conversion is about neutral or even slightly basic. At these conditions both carboxylic functions are negatively charged. One of them is balanced by the positively-charged ammonium group and the other by a cation, ammonium in most cases. This solution is acidulated through contact with a cation exchanger, the functional groups of which are at least partially in proton form. Due to the contact, cation exchange is effected, cations from the solution are adsorbed and protons are transferred from the cation exchanger to the solution, lowering its pH. Those protons react with one of the carboxylic groups on the aspartate (the one related to the pKa of 3.65) to form the zwitterion.

The solubility of the aspartic acid in the zwitterion form is low and its crystallization in the cation exchanger could damage the resin and interfere with its operation. Temperature elevation does not always solve the problem. Firstly, it is limited by the thermal stability of the resin and secondly the conditions within the resin pores are different from those in the free solution. Thus, according to JP94017346, in the case of contacting a solution-containing glutamic acid with a cation exchange resin, urea was added to avoid such crystallization. It was found that, in the case of the present process, acidulation by a cation exchanger can be effected without any significant problem resulting from crystallization of aspartic acid in the resin.

The acidulated solution is separated from the cation-carrying cation exchanger and the latter is sent to regeneration in contact with an acidic solution. The separation is preferably effected without cooling the solution much below the temperature of the acidulation step.

The cation exchanger carrying the adsorbed cations is contacted with a solution of the reagent acid or of its acidic salt. The reagent acid could be obtained in a petrochemical process, e.g. maleic acid or maleic anhydride, or from conversion of a petrochemical product, such as in the case of fumaric acid formation from maleic acid. In these cases, it is usually obtained in the free acid form. It could also be obtained through fermentation of a carbohydrate. Most of the fermentation of carbohydrates are conducted at about neutral pH and result in the neutral salt, e.g. diammonium fumarate. Such neutral salts could result from other processes, or from in a side stream of the main process. These neutral salts can be converted into the free acid, or to the acidic salt, e.g., monoammonium fumarate, by a variety of known methods such as water splitting electrodialysis or cation exchange. Acidic salt could result from other sources as well.

Maleic acid is one of the strongest carboxylic acids with a first pKa of 1.83. In contacting cation exchangers that carry adsorbed cations with a solution of maleic acid, a cation exchange reaction takes place. Protons from the aqueous solution are adsorbed on the resin, which is thereby regenerated and cations are released into the solution. In the case of very weak cation exchangers, both protons of the maleic acid can be exchanged (the second pKa of maleic acid is 6.07). In most cases, however, only one of the protons is exchanged. Maleic acid is strong enough to regenerate weak, moderate and even some strong acid cation exchangers. It provides, therefore, a large degree of freedom in selecting the cation exchanger for the present process.

The ability to conduct the process with some of the other reagent acids is surprising and, even more so, the ability to conduct it with their acidic salts. Aspartic acid's isoelectric point is highly acidic, 2.77, and one would expect that quite strong cation exchangers will be needed for its acidulation prior to the crystallization step. As a result, the regeneration of these cation exchangers would require a quite strong acid. It was surprisingly found that cation exchangers strong enough for acidulating aspartic acid can be regenerated with a solution of fumaric acid (pKa₁=3.03) and, even more surprisingly, by a solution of monoammonium fumarate (pKa₂=4.44).

The solution obtained on such exchange for the regeneration in step (e) is adjusted to form the feed solution to the conversion reaction. Thus, for example, in the case of regenerating the cation exchanger with maleic acid, only the pH and the ammonia to maleate molar-ration will require adjustment, if the reagent acid for the conversion step is maleic. (Possibly only ammonia addition will be required). If, on the other hand, fumaric is the reagent acid, the maleate salt will have to be converted into a fumarate salt, e.g. by methods described in US patent 4,560,653. After adjustment, the solution is fed to the reaction for converting the reagent acid to aspartate.

Unlike in the case of said US patent 4,560,653, the feed solution has not been recycled from the crystallization step and does not contain the impurities present in relatively large amounts therein. It is also practically free of aspartic acid and therefore avoids the inhibition effect described above.

The aspartic acid-containing solution obtained in the acidulation step is treated by known methods to crystallize aspartic acid therefrom. Such treatment could be a simple cooling of the solution from the temperature used in the acidulation step (which, in most cases, is higher than ambient). It could also comprise water evaporation.

The present process provides a possibility of high yield recovery of aspartic acid from the mother liquor by an additional step. In contacting this mother liquor with a strong acid cation exchanger, aspartic acid obtains an additional proton from the cation exchanger and transforms into the cationic form. As such it is efficiently bound from the mother liquor.

Thus, in a preferred embodiment, a strong acid cation exchanger in its acid form is contacted with the mother liquor of aspartic acid crystallization. The aspartic acid contained in that mother liquor is adsorbed. The cation exchanger resulting from that step is contacted with a solution of the aspartate salt obtained in the conversion step. As a result, the cation of the aspartate salt is bound on the resin and the adsorbed aspartic acid is released into the solution. The latter is separated and sent to aspartic acid crystallization as in step (d) or to further acidulation as in step (b) and the cation exchanger is regenerated with the reagent acid. For this preferred embodiment the reagent acid is preferably maleic acid.

Losses, as in present industrial practice, or undesired recycling of aspartic acid to the conversion reaction, as suggested in US patent 4,560,653, are avoided. As a result, there is no need to push the conditions in the crystallization step to maximum yield. The conditions there can be adjusted to those ensuring the highest product yield. In addition, if required, the bioconversion step could be operated at a relatively low salt concentration, if preferred for higher stability of the enzyme medium, without losing in the overall recovery yield.

Use of a sequence of a weak acid cation exchanger and a strong acid cation exchanger could be preferred in some circumstances. In that case, the aqueous solution containing the aspartate salt obtained in the conversion reaction is first contacted with the weak acid cation exchanger, and the resulting solution is contacted with the strong acid cation exchanger resulting from the contact with the mother liquor. The aqueous solution obtained in that contact, is sent to the aspartic acid crystallization. Regeneration of the resins is done in the following sequence: The maleic acid is first contacted with the strong acid cation exchanger and then with the weak acid one. This way no significant excess of maleic acid is needed.

The free mother liquor obtained contains most of the impurities resulting from the various sources. As it is essentially aspartic acid free, there is no need to recycle it to the reaction (directly or indirectly). Therefore, recycle of impurities and their build-up in the system are avoided.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Example 1.

A 5% ammonium aspartate resulting from bioconversion in an earlier stage is transferred, at 80C through a column containing Dowex MWC-1, a weak acid cation exchanger (WACE), sold by Dow. The pH of the incoming solution is nearly neutral. That of the outgoing solution is about 3. The latter solution is cooled to 15C and about 90% of the aspartic acid contained therein is crystallized out.

The mother liquor of the crystallization is transferred through a column containing Dowex CM-12, a strong acid cation exchanger (SACE), sold by Dow. More than 90% of the aspartic acid values in the mother liquor are adsorbed on the cation exchanger. The SACE is then treated with a solution of 10% ammonium aspartate resulting from bioconversion. Ammonium ions from that solution are adsorbed and practically al the aspartic acid bound in the previous step is released into the solution.

A solution containing 15% maleic acid is transferred through the ammonium carrying WACE and SACE from the previous stages. The cation exchangers are converted back to their acid form.

The maleate solution obtained is converted to fumarate solution by the addition of ammonium bromide and-ammonium persulfate and then heating with agitation to about 80C according to the method described in US 4,560,653.

The fumarate solution is anion exchanged and carbon treated and its pH is adjusted to 8.5. it is then treated with aspartase to form ammonium aspartate as described in US 4,460,653.

## Claims

1. A process for the production of crystalline aspartic acid comprising the steps of:
(a) reacting a feed solution containing anions of a dicarboxylic reagent acid, whereby the reagent acid is converted to aspartate ions, to form a product solution containing a dissociated aspartate salt;
(b) acidulating a solution of said dissociated aspartate salt resulting from said reaction in step (a) by contacting it with a cation exchanger, whereby cations of the aspartate salt are adsorbed and protons are released to the solution;
((c) separating the aqueous solution from the cation exchanger;
(d) crystallizing aspartic acid from said separated solution;
(e) regenerating said cation exchanger obtained in step (b) by contacting it with a solution comprising the reagent acid or an acidic salt thereof, whereby protons from the solution are adsorbed on the cation exchanger and cations adsorbed in step (b) are released to the solution; and
(f) using the solution obtained in step (e) as a constituent of said feed solution.

2. A process according to claim 1, wherein said reagent acid is a dicarboxylic acid having four carbon atoms.

3. A process according to claim 1, wherein said reagent acid is selected from the group consisting of fumaric and maleic acid.

4. A process according to claim 1, wherein the molar ratio of ammonium to the reagent acid in said feed solution is between 1.5 to 1 and 3 to 1.

5. A process according to claim 1, wherein said reaction is biologically catalyzed.

6. A process according to claim 1, wherein ammonium forms at least 90% of the cations in said product solution.

7. A process according to claim 1, wherein said cation exchanger is of weak to moderate strength.

8. A process according to claim 1, wherein said cations adsorbed in said step (b) are mainly ammonium ions.

9. A process according to claim 1, wherein said contacting with said cation exchanger is conducted at above ambient temperature.

10. A process according to claim 1, wherein said crystallization involves cooling.

11. A process according to claim 1, wherein aspartic acid left in the mother liquor of said crystallization is recovered by adsorbing on a strong acid cation exchanger.

12. A process according to claim 11, wherein said aspartic acid-carrying strong acid cation exchanger is contacted with a solution of an aspartate salt, cations of said salt are adsorbed and aspartic acid is released into the solution.

13. A process according to claim 12, wherein aspartic acid is crystallized out of said solution.

14. A process according to claim 11, wherein said strong acid cation exchanger carrying cations of said aspartate salt is contacted with a solution of said reagent acid, whereby protons from the solution are adsorbed on said cation exchanger and cations are released into the solution.

15. A process according to claim 14, wherein said solution formed is separated and used as a constituent of said feed solution.

16. A process according to claim 14, wherein said reagent acid is maleic acid.

17. A process according to claim 1, wherein said reagent acid is a fermentation product.

18. A process according to claim 1, wherein said reagent acid is maleic acid and said maleate-containing solution obtained in step (e) is converted to a fumarate-containing solution.

19. A process according to claim 1, wherein said contact with the cation exchanger in said step (b) is done in a counter-current mode.

20. A process according to claim 1, wherein said contact with the cation exchanger in said step (e) is done in a counter-current mode.

21. A process according to claim 1, wherein said acidic salt in step (e) is selected from a group consisting of monoammonium fumarate or monoammonium maleate.

22. A process according to claim 1, wherein at least 90% of said aspartate salt is acidulated in said step (b).

23. A process according to claim 1, wherein at least 98% of said aspartate salt is acidulated in said step (b).

## Patentansprüche

1. Verfahren für die Herstellung kristalliner Asparaginsäure, umfassend die Schritte:
(a) Umsetzen einer Ausgangslösung, die Anionen eines Dicarbonsäure-Reagens enthält, bei welcher das Säure-Reagens umgewandelt wird zu Aspartat-Ionen, um eine Produktlösung zu erzeugen, die ein dissoziiertes Aspartatsalz enthält;
(b) Ansäuern einer Lösung des dissoziierten Aspartatsalzes, resultierend aus der Reaktion in Schritt (a), indem sie mit einem Kationenaustauscher kontaktiert wird, wodurch Kationen des Aspartatsalzes adsorbiert und Protonen an die Lösung freigesetzt werden;
(c) Abtrennen der wässrigen Lösung von dem Kationenaustauscher;
(d) Auskristallisieren der Asparaginsäure aus der abgetrennten Lösung;
(e) Regenerieren des Kationenaustauschers, der in Schritt (b) erhalten wird, indem dieser mit einer Lösung kontaktiert wird, die das Säure-Reagens oder ein saures Salz davon aufweist, wodurch Protonen aus der Lösung auf dem Kationenaustauscher adsorbiert werden und in Schritt (b) adsorbierte Kationen in die Lösung freigesetzt werden; sowie
(f) Verwenden der in Schritt (e) erhaltenen Lösung als ein Bestandteil der Ausgangslösung.

2. Verfahren nach Anspruch 1, bei welchem das Säure-Reagens eine Dicarbonsäure ist, die vier Kohlenstoffatome hat.

3. Verfahren nach Anspruch 1, bei welchem das Säure-Reagens ausgewählt wird aus der Gruppe, bestehend aus Fumar- und Maleinsäure.

4. Verfahren nach Anspruch 1, bei welchem das Molverhältnis von Ammonium zu dem Säure-Reagens in der Ausgangslösung zwischen 1,5 zu 1 und 3 zu 1 liegt.

5. Verfahren nach Anspruch 1, bei welchem die Reaktion biologisch katalysiert wird.

6. Verfahren nach Anspruch 1, bei welchem Ammonium mindestens 90% der Kationen in der Produktlösung bildet.

7. Verfahren nach Anspruch 1, bei welchem der Kationenaustauscher eine schwache bis mittlere Konzentration hat.

8. Verfahren nach Anspruch 1, bei welchem die in dem Schritt (b) adsorbierten Kationen hauptsächlich Ammonium-Ionen sind.

9. Verfahren nach Anspruch 1, bei welchem das Kontaktieren mit dem Kationenaustauscher oberhalb der Umgebungstemperatur ausgeführt wird.

10. Verfahren nach Anspruch 1, bei welchem die Kristallisation ein Kühlen umfasst.

11. Verfahren nach Anspruch 1, bei welchem die in der Mutterlauge der Kristallisation zurückbleibende Asparaginsäure durch Adsorbieren an einem stark sauren Kationenaustauscher wiedergewonnen wird.

12. Verfahren nach Anspruch 11, bei welchem der die Asparaginsäure haltende, stark saure Kationenaustauscher kontaktiert wird mit einer Lösung eines Aspartatsalzes, wobei die Kationen des Salzes adsorbiert und Asparaginsäure in die Lösung freigesetzt wird.

13. Verfahren nach Anspruch 12, bei welchem Asparaginsäure aus dieser Lösung auskristallisiert wird.

14. Verfahren nach Anspruch 11, bei welchem der stark saure, die Kationen dieses Aspartatsalzes haltende Kationenaustauscher kontaktiert wird mit einer Lösung des Säure-Reagens, wodurch Protonen aus der Lösung an dem Kationenaustauscher adsorbiert und Kationen in die Lösung freigesetzt werden.

15. Verfahren nach Anspruch 14, bei welchem die gebildete Lösung abgetrennt und als Bestandteil der Ausgangslösung verwendet wird.

16. Verfahren nach Anspruch 14, bei welchem das Säure-Reagens Maleinsäure ist.

17. Verfahren nach Anspruch 1, bei welchem das Säure-Reagens ein Fermentationsprodukt ist.

18. Verfahren nach Anspruch 1, bei welchem das Säure-Reagens Maleinsäure ist und die in Schritt (e) erhaltene Maleat enthaltende Lösung umgewandelt wird zu einer Fumarat enthaltenden Lösung.

19. Verfahren nach Anspruch 1, bei welchem der Kontakt mit dem Kationenaustauscher in dem Schritt (b) in einer Gegenstrom-Betriebsweise ausgeführt wird.

20. Verfahren nach Anspruch 1, bei welchem der Kontakt mit dem Kationenaustauscher in dem Schritt (e) in einer Gegenstrom-Betriebsweise ausgeführt wird.

21. Verfahren nach Anspruch 1, bei welchem das saure Salz in Schritt (e) ausgewählt wird aus einer Gruppe, bestehend aus Monoammoniumfumurat oder Monoammoniummaleat.

22. Verfahren nach Anspruch 1, bei welchem mindestens 90% des Aspartatsalzes in dem Schritt (b) angesäuert werden.

23. Verfahren nach Anspruch 1, bei welchem mindestens 98% des Aspartatsalzes in dem Schritt (b) angesäuert werden.

## Revendications

1. Procédé pour la préparation d'acide aspartique cristallin, comprenant les étapes consistant:
(a) à faire réagir une solution d'alimentation contenant des anions d'un réactif acide dicarboxylique, de sorte que le réactif acide est converti en ions aspartate, pour former une solution de produit contenant un sel aspartate dissocié;
(b) à aciduler une solution dudit sel aspartate dissocié résultant de ladite réaction de l'étape (a) en la mettant en contact avec un échangeur de cations, de sorte que les cations du sel aspartate sont adsorbés et des protons sont libérés dans la solution;
(c) à séparer la solution aqueuse de l'échangeur de cations;
(d) à cristalliser l'acide aspartique à partir de ladite solution séparée;
(e) à régénérer ledit échangeur de cations obtenu dans l'étape (b) en le mettant en contact avec une solution comprenant le réactif acide ou un sel acide de celui-ci, de sorte que les protons de la solution sont adsorbés sur l'échangeur de cations et les cations adsorbés dans l'étape (b) sont libérés dans la solution; et
(f) à utiliser la solution obtenue dans l'étape (e) en tant que constituant de ladite solution d'alimentation.

2. Procédé selon la revendication 1, dans lequel ledit réactif acide est un acide dicarboxylique ayant quatre atomes de carbone.

3. Procédé selon la revendication 1, dans lequel ledit réactif acide est choisi dans le groupe constitué par les acides fumarique et maléique.

4. Procédé selon la revendication 1, dans lequel le rapport molaire d'ammonium au réactif acide dans ladite solution d'alimentation est entre 1,5 à 1 et 3 à 1.

5. Procédé selon la revendication 1, dans lequel ladite réaction est catalysée biologiquement.

6. Procédé selon la revendication 1, dans lequel l'ammonium forme au moins 90% des cations dans ladite solution de produit.

7. Procédé selon la revendication 1, dans lequel ledit échangeur de cations est de force faible à modérée.

8. Procédé selon la revendication 1, dans lequel lesdits cations adsorbés dans ladite étape (b) sont principalement des ions ammonium.

9. Procédé selon la revendication 1, dans lequel ladite mise en contact avec ledit échangeur de cations est effectuée au-dessus de la température ambiante.

10. Procédé selon la revendication 1, dans lequel ladite cristallisation fait intervenir un refroidissement.

11. Procédé selon la revendication 1, dans lequel l'acide aspartique restant dans la liqueur mère de ladite cristallisation est récupéré par adsorption sur un échangeur de cations acide fort.

12. Procédé selon la revendication 11, dans lequel ledit échangeur de cations acide fort portant de l'acide aspartique est mis en contact avec une solution d'un sel aspartate, les cations dudit sel sont adsorbés et l'acide aspartique est libéré dans la solution.

13. Procédé selon la revendication 12, dans lequel l'acide aspartique est cristallisé de ladite solution.

14. Procédé selon la revendication 11, dans lequel ledit échangeur de cations acide fort portant des cations dudit sel aspartate est mis en contact avec une solution dudit réactif acide, de sorte que les protons de la solution sont adsorbés sur ledit échangeur de cations et les cations sont libérés dans la solution.

15. Procédé selon la revendication 14, dans lequel ladite solution formée est séparée et utilisée en tant que constituant de ladite solution d'alimentation.

16. Procédé selon la revendication 14, dans lequel ledit réactif acide est l'acide maléique.

17. Procédé selon la revendication 1, dans lequel ledit réactif acide est un produit de fermentation.

18. Procédé selon la revendication 1, dans lequel ledit réactif acide est l'acide maléique et ladite solution contenant un maléate obtenue dans l'étape (e) est convertie en une solution contenant un fumarate.

19. Procédé selon la revendication 1, dans lequel ledit contact avec l'échangeur de cations dans ladite étape (b) est effectué en un mode à contre-courant.

20. Procédé selon la revendication 1, dans lequel ledit contact avec l'échangeur de cations dans ladite étape (e) est effectué en un mode à contre-courant.

21. Procédé selon la revendication 1, dans lequel ledit sel acide dans l'étape (e) est choisi dans le groupe constitué par le fumarate de monoammonium ou le maléate de monoammonium.

22. Procédé selon la revendication 1, dans lequel au moins 90% dudit sel aspartate est acidulé dans ladite étape (b).

23. Procédé selon la revendication 1, dans lequel au moins 98% dudit sel aspartate est acidulé dans ladite étape (b).
